(19) Europäisches Patentamt
European Patent Office
Office européen des brevets

(11) Veröffentlichungsnummer: **0 285 565 B1**

(12) **EUROPÄISCHE PATENTSCHRIFT**

(45) Veröffentlichungstag der Patentschrift: **15.09.93**

(51) Int. Cl.⁵: **C07D 413/04**, C07D 417/04, A01N 43/82

(21) Anmeldenummer: **88810194.6**

(22) Anmeldetag: **24.03.88**

# (54) 2-Mercapto-5-pyridyl-1,3,4-oxadiazole und -1,3,4-thiadiazole, Verfahren zu ihrer Herstellung und ihre Verwendung als nematizide Mittel.

(30) Priorität: **03.04.87 CH 1293/87**

(43) Veröffentlichungstag der Anmeldung:
**05.10.88 Patentblatt 88/40**

(45) Bekanntmachung des Hinweises auf die Patenterteilung:
**15.09.93 Patentblatt 93/37**

(84) Benannte Vertragsstaaten:
**AT BE CH DE ES FR GB GR IT LI LU NL**

(56) Entgegenhaltungen:
**EP-A- 0 127 847** **EP-A- 0 217 747**
**WO-A-86/07590** **DE-A- 2 361 613**
**DE-A- 2 853 196** **US-A- 3 770 754**

**CHEMICAL ABSTRACTS, Band 78, Nr. 7, 19. Februar 1973, Columbus, Ohio, USA, Seite 494, Spalte 1, abstract 43486r**

**CHEMICAL ABSTRACTS, Band 74, Nr. 17, 26. April 1971, Columbus, Ohio, USA, Seite 440, Spalte 2, abstract 87996d**

**CHEMICAL ABSTRACTS, Band 105, Nr. 25, 22. Dezember 1986, columbus, Ohio, USA , Seite 780, Spalte 2, Abstract 226453y**

**CHEMICAL ABSTRACTS, band 66, Nr. 15, 10. April 1967, Columbus, Ohio, USA, Seite 6157, Spalte 2, Abstract 65476s**

(73) Patentinhaber: **CIBA-GEIGY AG**
**Klybeckstrasse 141**
**CH-4002 Basel(CH)**

(72) Erfinder: **Nyfeler, Robert, Dr.**
**Bärenfelserstrasse 8**
**CH-4057 Basel(CH)**
Erfinder: **Eckhardt, Wolfgang, Dr.**
**Breslauerstrasse 14**
**D-7850 Lörrach(DE)**
Erfinder: **Beriger, Ernst, Dr.**
**Grabenmattweg 29**
**CH-4123 Allschwil(CH)**
Erfinder: **Kristiansen, Odd, Dr.**
**Delligrabenstrasse 7**
**CH-4313 Möhlin(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue substituierte 2-Mercapto-5-pyridyl-1,3,4-oxadiazole und 2-Mercapto-5-pyridyl-1,3,4-thiadiazole, deren Herstellung sowie nematizide Mittel, die als Wirkstoff mindestens eine dieser Verbindungen enthalten. Die Erfindung betrifft ferner die Verwendung von 2-Mercapto-5-pyridyl-1,3,4-oxadiazolen und von 2-Mercapto-5-pyridyl-1,3,4-thiadiazolen und Mittel zur Bekämpfung von Nematoden, insbesondere von pflanzenschädigenden Nematoden.

Die erfindungsgemässen 2-Mercapto-5-pyridyl-1,3,4-oxadiazole und 2-Mercapto-5-pyridyl-1,3,4-thiadiazole entsprechen der allgemeinen Formel I

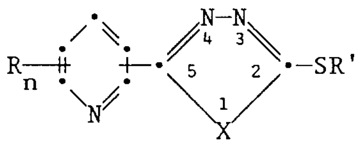

(I)

in welcher

X Sauerstoff oder Schwefel

R' Difluormethyl

R $C_1$-$C_3$-Alkyl, unsubstituiertes oder durch Halogen oder $C_1$-$C_3$-Alkoxy substituiertes $C_1$-$C_3$-Alkoxy; unsubstituiertes oder durch Halogen substituiertes $C_3$-$C_7$-Alkenyl; $C_3$-$C_7$-Alkinyl, $C_1$-$C_3$-Alkylthio, Halogen, Cyano, Hydroxy, unsubstituiertes oder durch $C_1$-$C_3$-Alkyl mono- oder disubstituiertes Amino; oder Aminocarbonyl bedeuten und

n für 0, 1, 2, 3 oder 4 steht,

sowie den Salzen einer dieser Verbindungen.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die Propyl- und Isopropylgruppe. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Fluor, Chlor oder Brom.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Weiterhin sind in WO-A-86/07590 1,3,4-Oxadiazole und 1,3,4-Thiadiazole als Nematizide beschrieben, die sich von den erfindungsgemässen Verbindungen dadurch unterscheiden, dass sie nicht durch Pyridyl und Difluormethylmercapto substituiert sind, sondern u.a. durch Phenyl beziehungsweise $F_2C=CF(CH_2)_n$-S- (n = 1-4). Diese bekannten Verbindungen haben bisher als Nematizide die in der Praxis an sie gestellten Ansprüche nicht in vollem Umfang befriedigen können. Ferner sind Oxadiazol-Derivate mit fungizider Wirksamkeit in der DE-OS 2 361 613 beschrieben. Es werden darin jedoch keine dieser Verbindungen expressis verbis genannt, die unter den Umfang der erfindungsgemässen Formel I fallen, und eine nematizide Wirkung ist dort nicht beschrieben.

Durch die Bereitstellung der erfindungsgemässen Verbindungen der Formel I ist es nun gelungen einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen wie z.B. Kartoffeln, Getreide, Rüben, Raps, Kohl, Tabak, Sojabohnen, Baumwolle und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus,

Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht. Die nematizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Im Rahmen der vorliegenden Erfindung sind folgende Verbindungsgruppen und Einzelverbindungen der Formel I bevorzugt:

1. Jene 2-Mercapto-5-(pyrid-2-yl)-1,3,4-oxadiazole der Formel Ia,

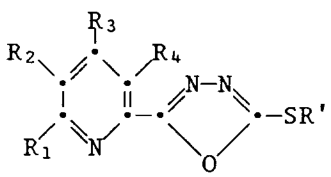

(Ia) ,

in welchen R' Difluormethyl, die Reste $R_1$-$R_3$ Wasserstoff und $R_4$ Aminocarbonyl oder Hydroxy bedeutet.

1.1 Von diesen Verbindungen ist das 2-Difluormethylthio-5-(pyrid-2-yl)-1,3,4-oxadiazol besonders hervorzuheben.

2. Jene 2-Mercapto-5-(pyrid-2-yl)-thiadiazole der Formel Ib

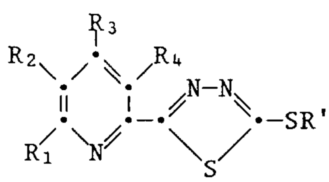

(Ib) ,

in welchen R' Difluormethyl; $R_1$-$R_4$ voneinander unabhängig Wasserstoff, Methyl, Methoxy, Methylmercapto, Chlor, Cyano, Hydroxy, Amino oder Aminocarbonyl bedeuten.

Von diesen Verbindungen sind jene insbesondere bevorzugt, in welchen

2.1 $R_1$ und $R_4$ voneinander unabhängig Wasserstoff oder Chlor, $R_2$ und $R_3$ Wasserstoff bedeuten, ferner jene, in welchen

2.2 $R_1$, $R_2$ und $R_3$ Wasserstoff und $R_4$ Methyl, Methoxy, Chlor, Cyano, Aminocarbonyl Hydroxy oder Amino bedeuten, sowie jene, in welchen

2.3 einer der Reste $R_1$, $R_2$ und $R_3$ Methyl, Chlor, Methoxy oder Methylmercapto, die restlichen zwei Reste Wasserstoff und $R_4$ Wasserstoff bedeuten, und schliesslich jene, in welchen

2.4 $R_1$ und $R_4$ voneinander unabhängig Wasserstoff, Chlor, Methoxy oder Hydroxy und $R_2$ und $R_3$ Wasserstoff bedeuten.

2.4.1 Von der letztgenannten Gruppe ist das 2-Difluormethylthio-5-(pyrid-2-yl)-1,3,4-thiadiazol hervorzuheben.

3.1 Jene 2-Mercapto-5-(pyrid-3-yl)oxadiazole der Formel Ic

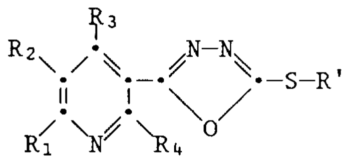

(Ic) ,

in welchen

R' Difluormethyl und einer der Reste $R_1$-$R_4$ Methyl oder Methylmercapto und die restlichen drei Reste Wasserstoff bedeuten, ferner jene, in welchen

3.2 R' Difluormethyl und zwei der Reste $R_1$ und $R_4$ voneinander unabhängig Methyl oder Chlor und $R_2$ und $R_3$ Wasserstoff bedeuten,

3.3 das Difluormethylthio-5-(pyrid-3-yl)-1,3,4-oxadiazol.

4.1 Jene 2-Mercapto-5-(pyrid-3-yl)-thiadiazole der Formel Id

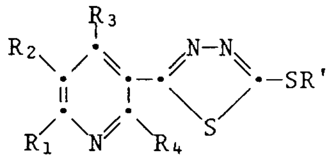

(Id) ,

in welchen

R' Difluormethyl und $R_1$-$R_4$ Wasserstoff bedeuten, ferner jene, in welchen

4.2 R' Difluormethyl, $R_1$ Wasserstoff, $C_1$-$C_3$-Alkyl oder Chlor, $R_2$ Wasserstoff und $R_3$ und $R_4$ voneinander unabhängig Wasserstoff oder Chlor oder Methoxy bedeuten, schliesslich jene, in welchen

4.3 R' Difluormethyl, $R_1$, $R_2$ und $R_4$ voneinander unabhängig Wasserstoff, Chlor, Brom, Methylthio oder Amino und $R_3$ Wasserstoff bedeuten.

5. Jene 2-Mercapto-5-(pyrid-4-yl)-oxadiazole der Formel Ie

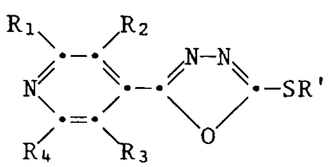

(Ie) ,

in welchen

R' Difluormethyl und $R_1$-$R_4$ Wasserstoff bedeuten.

6. Jene 2-Mercapto-5-(pyrid-4-yl)-thiadiazole der Formel If

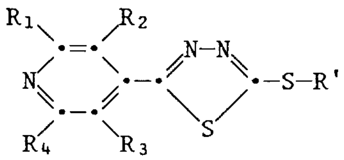

(If) ,

in welchen

R' Difluormethyl, $R_1$ Wasserstoff, $C_1$-$C_3$-Alkyl, Chlor, Methoxy oder Methylmercapto, $R_4$ Wasserstoff, Methyl, Chlor, Methoxy-äthoxy, Trifluormethylmethoxy, Methylmercapto, Amino oder $C_1$-$C_3$-Monoalkylamino, und $R_2$ und $R_3$ Wasserstoff bedeuten.

6.2 Von der letztgenannten Gruppe ist das 2-Difluormethylthio-5-(pyrid-4-yl)-1,3,4-thiadiazol hervorzuheben.

Die Verbindungen der Formel I werden erfindungsgemäss hergestellt, indem man in einer Kondensationsreaktion eine Verbindung der Formel II

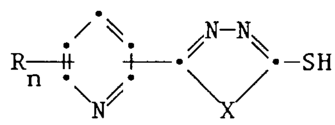

(II)

oder eine Verbindung der Formel III

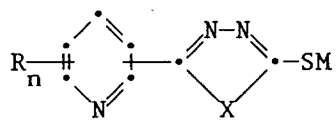

(III)

mit einer Verbindung der Formel IV

Hal-R' (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, wobei in den vorstehend genannten Formeln II, III und IV M für ein Alkalimetall oder für Ammonium steht, Hal Halogen, vorzugsweise Chlor, Brom oder Jod bedeutet, während R, R', X und n die unter Formel I angegebenen Bedeutungen besitzen.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkyli- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriumethylat.

Die Zugabe katalytischer Mengen eines Kronenethers, wie z.B. 18-Krone-6 oder 15-Krone-5, wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylammoniumchloride oder -bromide, vorzugsweise Tetra-n-butylammoniumbromid, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10 bis 90 °C, vorzugsweise 30 ° bis 80 °C. Das Verfahren wird unter atmospherischem oder gegebenenfalls unter erhöhtem Druck durchgeführt.

Die Ausgangsverbindungen der Formel II sind teils bekannt teils neu. Neu sind davon jene 2-Mercapto-5-pyrid-2-yl-1,3,4-oxadiazole der Formel IIa'

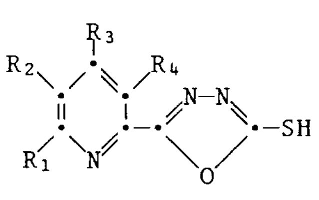

(IIa'),

2-Mercapto-5-pyrid-2-yl-1,3,4-thiadiazole der Formel IIb'

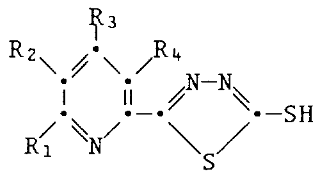

(IIb'),

2-Mercapto-5-pyrid-3-yl-1,3,4-oxadiazole der Formel IIc'

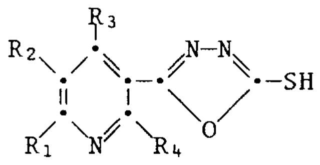

(IIc'),

2-Mercapto-5-pyrid-3-yl-1,3,4-thiadiazole der Formel IId'

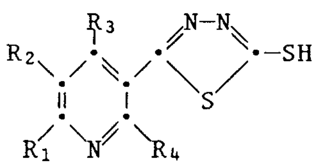

(IId'),

2-Mercapto-5-pyrid-4-yl-1,3,4-oxadiazole der Formel IIe'

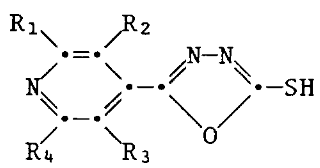

(IIe')

und 2-Mercapto-5-pyrid-4-yl-1,3,4-thiadiazole der Formel IIf'

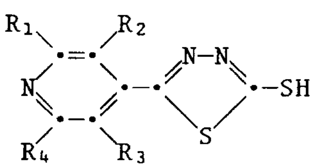

(IIf')

in welchen jeweils $R_1$-$R_4$ voneinander unabhängig Methyl, Propyl oder Isopropyl, unsubstituiertes oder durch Halogen oder $C_1$-$C_3$-Alkoxy substituiertes $C_1$-$C_3$-Alkoxy; unsubstituiertes oder durch Halogen substituiertes $C_3$-$C_7$-Alkenyl; $C_4$-$C_7$-Alkinyl, $C_1$-$C_3$-Alkylthio, Halogen, Cyano, Hydroxy, unsubstituiertes oder durch $C_1$-$C_3$-Alkyl mono- oder disubstituiertes Amino; oder Aminocarbonyl, bedeuten.

Die neuen Verbindungen der Formeln IIa'-IIf' (siehe Tabelle 0) sind Zwischenprodukte zur Herstellung wertvoller nematizider Wirkstoffe und bilden damit einen Bestandteil der vorliegenden Erfindung.

Die Ausgangsverbindungen der Formel II können nach bekannten Methoden [Salama et al: Egypt. J. Chem. 1981 (Pub. 1982) 24 (1-3) 47-51 CA. 99, 53674 y (1983): Pol. J. Pharmacol. Pharm. 1981, 33(5), 527-32, CA. 97, 6229 f, 1982; Chem. Pharm. Bull. 1970, 18(8), 1696-8, CA. 73, 98877 w, 1970] oder in Analogie zu ihnen hergestellt werden.

Die Verbindungen der Formel IV sind bekannte, im Handel erhältliche Produkte.

Die Erfindung betrifft auch Mittel, zur Bekämpfung von pflanzenschädigenden Nematoden sowie zur präventiven Verhütung des Nematodenbefalls von Pflanzgut, welche die Wirkstoffe der Formel I enthalten.

Darüber hinaus schliesst die vorliegende Erfindung zusätzlich die Herstellung nematizider Mittel ein, die gekennzeichnet ist durch das innige Vermischen von Aktivsubstanzen der Formel I mit einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel I,

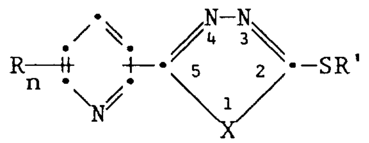

(I)

in welcher

X Sauerstoff oder Schwefel

R' Difluormethyl

R Wasserstoff, $C_1$-$C_3$-Alkyl, unsubstituiertes oder durch Halogen oder $C_1$-$C_3$-Alkoxy substituiertes $C_1$-$C_3$-Alkoxy; unsubstituiertes oder durch Halogen substituiertes $C_3$-$C_7$-Alkenyl; $C_3$-$C_7$-Alkinyl, $C_1$-$C_3$-Alkylthio, Cyano, Hydroxy, unsubstituiertes oder durch $C_1$-$C_3$-Alkyl mono- oder disubstituiertes Amino; oder Aminocarbonyl,

n für 0, 1, 2, 3 oder 4 steht,

oder der neuen Mittel charakterisiert ist.

Ein bezvorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I" nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsauren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die neuen Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung:

1. Herstellungsbeispiele

Herstellungsbeispiel 0.4

2-Mercapto-5-(pyrid-3-yl)-1,3,4-oxadiazol

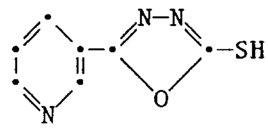

Zur vorgelegten Lösung von 56 g (0,1 Mol) Kaliumhydroxid in 20 g Wasser und 350 ml 95%-igem Aethanol werden 13,7 g Nicotinsäurehydrazid (0,1 Mol) und 8 g (0,105 Mol) Schwefelkohlenstoff getropft. Das Reaktionsgut wird 3 Stunden lang unter Rückfluss gekocht. Das Lösungsmittel wird anschliessend in Vakuum verdampft der Rückstand in Hochvakuum getrocknet. Der trockene Rückstand wird in 75 ml Wasser angerührt, filtriert und das Filtrat mit 1 N Salzsäure neutral gestellt. Das ausfallende Produkt wird abfiltriert, nacheinander mit Aceton und Hexan nachgewaschen und im Vakuum getrocknet. Man erhielt so 9,9 g (55 % d. Th.) vom Smp. 237 ° C.

Herstellungsbeispiel 1.1.

2-Difluormethylthio-5-pyrid-2-yl-1,3,4-thiadiazol

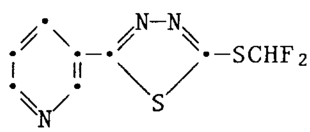

Zu einer Lösung von 230,5 g 85%-igem Kaliumhydroxid in 800 ml Wasser wird eine Anschlämmung von 341,5 g (1,75 Mol) 2-Mercapto-5-pyrid-2-yl-1,3,4-thiadiazol in 4000 ml Dioxan gegeben. Anschliessend werden 10 g Kaliumjodid und 6 g Tetrabutylammoniumbromid zum Reaktionsgemisch gegeben und während 4,5 Stunden bei 22 bis 37 ° C 750 g (~8,6 Mol) gasförmiges Difluormethan eingeleitet. Nach Verdampfen des Dioxans wird das Reaktionsgemisch mit Essigsäureethylester verdünnt, die Wasserphase abgetrennt und die organische Lösung mit 1N Natronlauge in der Kälte und Wasser gewaschen, mit Natriumsulfat getrocknet, mit Aktivkohle geklärt und der Essigsäureethylester im Vakuum verdampft. Der Rückstand wird aus Essigsäureethylester/Hexan kristallisiert. Man erhält so 321,9 g (75,1 %) Produkt vom Smp. 90-92 ° C.

Analog der vorstehenden Herstellungsbeispiele sowie der vorbeschriebenen Verfahren lassen sich folgende erfindungsgemässe Verbindungen herstellen. Die nachfolgend aufgeführten Verbindungen dienen der Illustration der vorliegenden Erfindung.

Tabelle 0

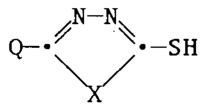

| Nr. | Q | X | Physik. Daten |
|---|---|---|---|
| 0.1 | 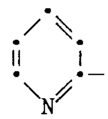 | -S- | Smp. >200°C |
| 0.2 | 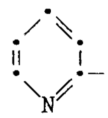 | -O- | Smp. >200°C |
| 0.3 | 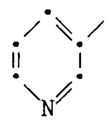 | -S- | Smp. 276-279°C |
| 0.4 | 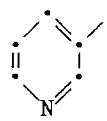 | -O- | Smp. 237°C |
| 0.5 | 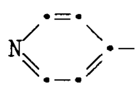 | -S- | Smp. >200°C |
| 0.6 | 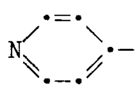 •HCl | -O- | Smp. >200°C |
| 0.7 | 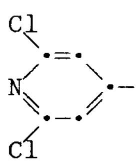 | -S- | Smp. 192-194°C |
| 0.8 | 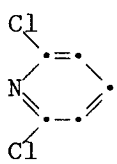 | -O- | Smp. 202-205°C |
| 0.9 | 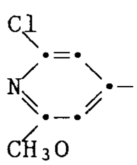 | -S- | Smp. 215-218°C |

Tabelle 0 (Fortsetzung)

| Nr. | Q | X | Physik. Daten |
| --- | --- | --- | --- |
| 0.10 | 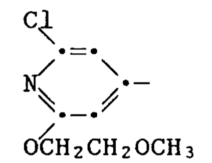 | -S- | Smp. 153-156°C |
| 0.11 | 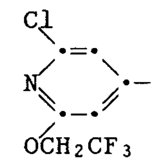 | -S- | |
| 0.12 | 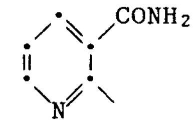 | -S- | |
| 0.13 | 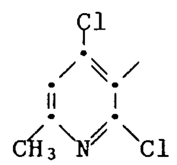 | -S- | |
| 0.14 | 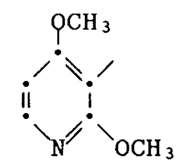 | -S- | |
| 0.15 | 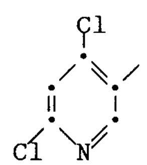 | -S- | |
| 0.16 | 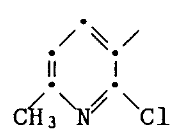 | -S- | |
| 0.17 | 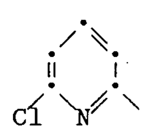 | -S- | |
| 0.18 | 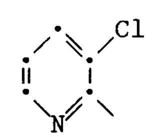 | -S- | |

Tabelle 0 (Fortsetzung)

| Nr. | Q | X | Physik. Daten |
|---|---|---|---|
| 0.19 | 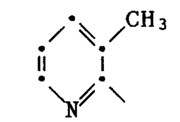 | -S- | |
| 0.20 | 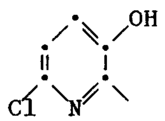 | -S- | |
| 0.21 | 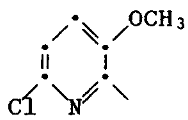 | -S- | |
| 0.22 | 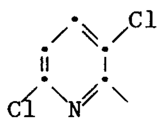 | -S- | |
| 0.23 | 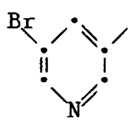 | -S- | Smp. >200°C |
| 0.24 | 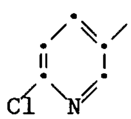 | -S- | |
| 0.25 | 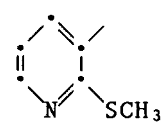 | -S- | Smp. >200°C |
| 0.26 | 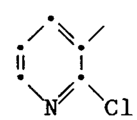 | -S- | |
| 0.27 | 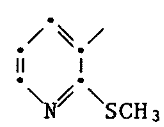 | -O- | Smp. >200°C |
| 0.28 | 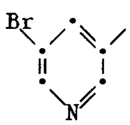 | -O- | Smp. >200°C |
| 0.29 | 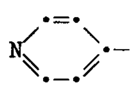 | -O- | Smp. >200°C |

Tabelle 0 (Fortsetzung)

| Nr. | Q | X | Physik. Daten |
|---|---|---|---|
| 0.30 | 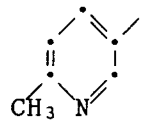 | -S- | Smp. >250°C |
| 0.31 | 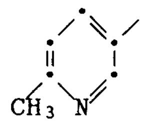 | -O- | Smp. >250°C |

Tabelle 1

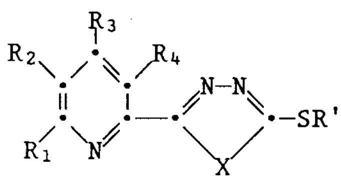

| Verb. Nr. | R' | $R_1$ | $R_2$ | $R_3$ | $R_4$ | X | Physikal. Daten |
|---|---|---|---|---|---|---|---|
| 1.1 | $-CHF_2$ | H- | H- | H- | -H | -S- | Smp. 90-92°C |
| 1.2 | $-CHF_2$ | H- | H- | H- | -H | -O- | Smp. 82-84°C |
| 1.3 | $-CHF_2$ | $CH_3-$ | H- | H- | -H | -S- | |
| 1.4 | $-CHF_2$ | H- | $CH_3-$ | H- | -H | -S- | |
| 1.5 | $-CHF_2$ | H- | H- | $CH_3-$ | -H | -S- | |
| 1.6 | $-CHF_2$ | H- | H- | H- | $-CH_3$ | -S- | |
| 1.7 | $-CHF_2$ | Cl- | H- | H- | -H | -S- | |
| 1.8 | $-CHF_2$ | H- | H- | H- | -Cl | -S- | |
| 1.9 | $-CHF_2$ | H- | H- | H- | -CN | -S- | |
| 1.10 | $-CHF_2$ | H- | H- | H- | $-CONH_2$ | -S- | |
| 1.11 | $-CHF_2$ | H- | H- | H- | $-CONH_2$ | -O- | |
| 1.12 | $-CHF_2$ | H- | H- | $OCH_3-$ | -H | -S- | |
| 1.13 | $-CHF_2$ | $CH_3O-$ | H- | H- | -H | -S- | |
| 1.14 | $-CHF_2$ | H- | Cl- | H- | -H | -S- | |
| 1.15 | $-CHF_2$ | H- | H- | Cl- | -H | -S- | |
| 1.16 | $-CHF_2$ | Cl- | H- | H- | -Cl | -S- | |
| 1.17 | $-CHF_2$ | Cl- | H- | H- | -Cl | -O- | |
| 1.18 | $-CHF_2$ | Cl- | H- | H- | $-OCH_3$ | -S- | |
| 1.19 | $-CHF_2$ | Cl- | H- | H- | -OH | -S- | |
| 1.20 | $-CHF_2$ | H- | H- | H- | -OH | -S- | |
| 1.21 | $-CHF_2$ | H- | H- | H- | -OH | -O- | |
| 1.22 | $-CHF_2$ | H- | H- | H- | $-OCH_3$ | -S- | |
| 1.23 | $-CHF_2$ | H- | H- | H- | $-NH_2$ | -S- | |
| 1.24 | $-CHF_2$ | $CH_3S-$ | H- | H- | -H | -S- | |
| 1.25 | $-CHF_2$ | H- | H- | H- | -J | -O- | |
| 1.26 | $-CHF_2$ | H- | H- | H- | -J | -S- | |

Tabelle 2

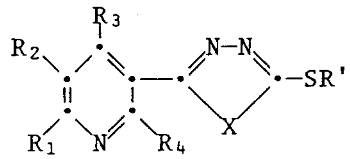

| Verb. Nr. | R' | $R_1$ | $R_2$ | $R_3$ | $R_4$ | X | Physikal. Daten |
|---|---|---|---|---|---|---|---|
| 2.1 | $-CHF_2$ | H- | H- | H- | -H | -S- | Smp. 86°C |
| 2.2 | $-CHF_2$ | H- | H- | H- | -H | -O- | $n_D^{20}$: 1,5715 |
| 2.3 | $-CHF_2$ | $CH_3-$ | H- | H- | -Cl | -S- | |
| 2.4 | $-CHF_2$ | $CH_3-$ | H- | H- | -Cl | -O- | |
| 2.5 | $-CHF_2$ | Cl- | H- | Cl- | -H | -S- | |
| 2.6 | $-CHF_2$ | Cl- | H- | Cl- | -H | -O- | |
| 2.7 | $-CHF_2$ | H- | H- | $OCH_3-$ | $-OCH_3$ | -S- | |
| 2.8 | $-CHF_2$ | $CH_3-$ | H- | Cl- | -Cl | -S- | |
| 2.9 | $-CHF_2$ | $CH_3-$ | H- | Cl- | -Cl | -O- | |
| 2.10 | $-CHF_2$ | Cl- | H- | H- | -Cl | -S- | |
| 2.11 | $-CHF_2$ | Cl- | H- | H- | -Cl | -O- | |
| 2.12 | $-CHF_2$ | $C_3H_7(n)-$ | H- | H- | -Cl | -S- | |
| 2.13 | $-CHF_2$ | Cl- | Cl- | H- | -Cl | -S- | |
| 2.14 | $-CHF_2$ | Cl- | Cl- | H- | -Cl | -O- | |
| 2.15 | $-CHF_2$ | H- | Br- | H- | $-NH_2$ | -S- | |
| 2.16 | $-CHF_2$ | H- | H- | H- | $-NH_2$ | -S- | |
| 2.17 | $-CHF_2$ | H- | Br- | H- | -H | -S- | Smp. 125-127°C |
| 2.18 | $-CHF_2$ | H- | Br- | H- | -H | -O- | Smp. 100-101°C |
| 2.19 | $-CHF_2$ | Cl- | H- | H- | -H | -S- | |
| 2.20 | $-CHF_2$ | Cl- | H- | H- | -H | -O- | |
| 2.21 | $-CHF_2$ | H- | H- | H- | $-SCH_3$ | -S- | Smp. 69-71°C |
| 2.22 | $-CHF_2$ | H- | H- | H- | $-SCH_3$ | -O- | Smp. 58-60°C |
| 2.23 | $-CHF_2$ | $CH_3-$ | H- | H- | -H | -S- | |
| 2.24 | $-CHF_2$ | $CH_3-$ | H- | H- | -H | -O- | |

Tabelle 3

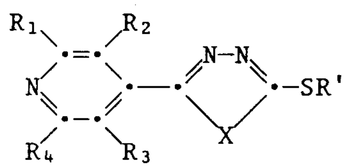

| Verb. Nr. | R' | $R_1$ | $R_2$ | $R_3$ | $R_4$ | X | Physikal. Daten |
|---|---|---|---|---|---|---|---|
| 3.1 | $-CHF_2$ | H- | H- | H- | H | -S- | Smp. 87-90°C |
| 3.2 | $-CHF_2$ | Cl- | H- | H- | -Cl | -S- | Smp. 59-62°C |
| 3.3 | $-CHF_2$ | Cl- | H- | H- | -Cl | -O- | |
| 3.4 | $-CHF_2$ | $CH_3$- | H- | H- | -Cl | -S- | |
| 3.5 | $-CHF_2$ | Cl- | H- | H- | -H | -S- | |
| 3.6 | $-CHF_2$ | $OCH_3$- | H- | H- | -H | -S- | |
| 3.7 | $-CHF_2$ | $CH_3$- | H- | H- | -H | -S- | |
| 3.8 | $-CHF_2$ | $OCH_3$ | H- | H- | -Cl | -S- | Smp. 82-85°C |
| 3.9 | $-CHF_2$ | Cl- | H- | H- | $-OCH_2CH_2OCH_3$ | -S- | Smp. 76-79°C |
| 3.10 | $-CHF_2$ | Cl- | H- | H- | $-OCH_2CF_3$ | -S- | Oel |
| 3.11 | $-CHF_2$ | $n-C_3H_7$- | H- | H- | -Cl | -S- | |
| 3.12 | $-CHF_2$ | Cl- | H- | H- | $-NHCH_3$ | -S- | |
| 3.13 | $-CHF_2$ | Cl- | H- | H- | -OH | -S- | |
| 3.14 | $-CHF_2$ | Cl- | H- | H- | $-NH_2$ | -S- | |
| 3.15 | $-CHF_2$ | Cl- | H- | H- | $-NHC_2H_5$ | -S- | |
| 3.16 | $-CHF_2$ | Cl- | H- | H- | $-NHC_3H_7(i)$ | -S- | |
| 3.17 | $-CHF_2$ | $CH_3$- | H- | H- | $-CH_3$ | -S- | |
| 3.18 | $-CHF_2$ | $SCH_3$- | H- | H- | $-SCH_3$ | -S- | |
| 3.19 | $-CHF_2$ | H- | H- | H- | -H | -O- | Smp. 88-93°C |

2. Formulierungsbeispiele für flüssige Wirkstoffe der Formel I (% = Gewichtsprozent)

2.1 Emulsions-Konzentrate

| | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

2.2 Lösungen

| | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 ° C) | - | - | 94 % | - |
| (MG = Molekulargewicht) | | | | |

Die Lösungen sind zur Anwendung in Form kleinster Tropfen geeignet.

2.3 Granulate

| | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Stäubemittel

| | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

2.5 Spritzpulver

| | a) | b) | c) |
|---|---|---|---|
| Wirkstoff aus den Tabellen 1-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Emulsions-Konzentrat

| | |
|---|---|
| Wirkstoff aus den Tabellen 1-3 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.7 Stäubemittel

| | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

2.8 Extruder Granulat

| | |
|---|---|
| Wirkstoff aus den Tabellen 1-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.9 Umhüllungs-Granulat

| | |
|---|---|
| Wirkstoff aus den Tabellen 1-3 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

| | |
|---|---|
| Wirkstoff aus den Tabellen 1-3 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

3.1 Wirkung gegen Meloidogyne incognita auf Tomaten

Eier von Meloidogyne incognita werden in Sand eingemischt. Dieses Gemisch wird dann in 200 ml fassende Töpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26±1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Verbindungen aus den Tabellen 1-3 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmt z.B. die Verbindung Nr. 1.1 im obigen Versuch die Wurzelgallbildung fast vollständig (0-10 % Restbefall).

**Patentansprüche**

**Patentansprüche für folgende Vertragsstaaten : AT, BE, CH, DE, FR, GB, GR, IT, LI, LU, NL**

**1.** 2-Mercapto-5-pyridyl-1,3,4-oxadiazole und 2-Mercapto-5-pyridyl-1,3,4-thiadiazole der Formel I

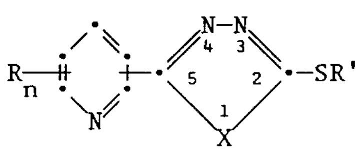

(I),

in welcher

- X Sauerstoff oder Schwefel,
- R' Difluormethyl und
- R $C_1$-$C_3$-Alkyl, unsubstituiertes oder durch Halogen oder $C_1$-$C_3$-Alkoxy substituiertes $C_1$-$C_3$-Alkoxy; unsubstituiertes oder durch Halogen substituiertes $C_3$-$C_7$-Alkenyl; $C_3$-$C_7$-Alkinyl, $C_1$-$C_3$-Alkylthio, Halogen, Cyano, Hydroxy, unsubstituiertes oder durch $C_1$-$C_3$-Alkyl mono- oder disubstituiertes Amino; oder Aminocarbonyl bedeuten und
- n für 0, 1, 2, 3 oder 4 steht, in freier Form oder in Salzform.

**2.** 2-Mercapto-5-(pyrid-2-yl)-1,3,4-oxadiazole gemäss Anspruch 1 der Formel Ia

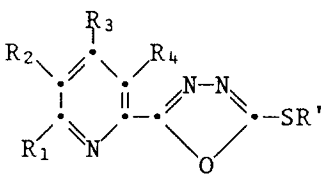

(Ia),

worin $R^1$ Difluormethyl, die Reste $R_1$-$R_3$ Wasserstoff und $R_4$ Aminocarbonyl oder Hydroxy bedeuten.

**3.** 2-Mercapto-5-(pyrid-2-yl)-1,3,4-oxadiazol gemäss Anspruch 1 der Formel Ia

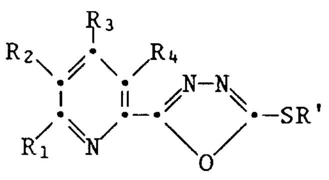

(Ia),

worin R' Difluormethyl und $R_1$-$R_4$ Wasserstoff bedeuten.

**4.** 2-Mercapto-5-(pyrid-2-yl)-1,3,4-thiadiazole gemäss Anspruch 1 der Formel Ib

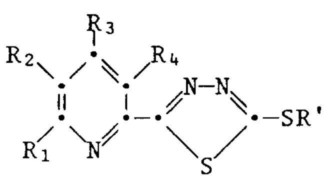

(Ib),

worin R' Difluormethyl und $R_1$-$R_4$ voneinander unabhängig Wasserstoff, Methyl, Methoxy, Methylmercapto, Chlor, Cyano, Hydroxy, Amino oder Aminocarbonyl bedeuten.

**5.** Verbindungen gemäss Anspruch 4 der Formel Ib, worin $R_1$ und $R_4$ voneinander unabhängig Wasserstoff oder Chlor und $R_2$ und $R_3$ Wasserstoff bedeuten.

**6.** Verbindungen gemäss Anspruch 4 der Formel Ib, worin R' Difluormethyl, $R_1$, $R_2$ und $R_3$ Wasserstoff und $R_4$ Methyl, Methoxy, Chlor, Cyano, Aminocarbonyl, Hydroxy oder Amino bedeuten.

**7.** Verbindungen gemäss Anspruch 4 der Formel Ib, worin R' Difluormethyl, einer der Reste $R_1$, $R_2$ und $R_3$ Methyl, Chlor, Methoxy oder Methylmercapto, die restlichen zwei Reste Wasserstoff und $R_4$ Wasserstoff bedeuten.

**8.** Verbindungen gemäss Anspruch 4 der Formel Ib, worin R' Difluormethyl, $R_1$ und $R_4$ voneinander unabhängig Wasserstoff, Chlor, Methoxy oder Hydroxy und $R_2$ und $R_3$ Wasserstoff bedeuten.

**9.** 2-Difluormethylthio-5-(pyrid-2-yl)-1,3,4-thiadiazol gemäss Anspruch 8.

**10.** 2-Mercapto-5-(pyrid-3-yl)-1,3,4-oxadiazole gemäss Anspruch 1 der Formel Ic

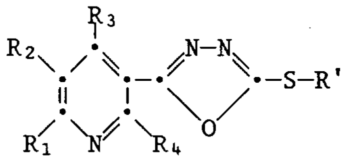

(Ic),

worin R' Difluormethyl und einer der Reste $R_1$-$R_4$ Methyl oder Methylmercapto und die restlichen drei Reste Wasserstoff bedeuten.

**11.** 2-Mercapto-5-(pyrid-3-yl)-1,3,4-oxadiazole gemäss Anspruch 1 der Formel Ic

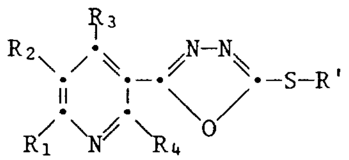

(Ic),

worin R' Difluormethyl, $R_1$ und $R_4$ voneinander unabhängig Methyl oder Chlor und $R_2$ und $R_3$ Wasserstoff bedeuten.

**12.** 2-Mercapto-5-(pyrid-3-yl)-1,3,4-oxadiazol gemäss Anspruch 1 der Formel Ic

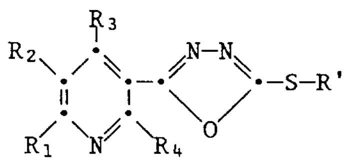

(Ic),

worin R' Difluormethyl und $R_1$-$R_4$ Wasserstoff bedeuten.

**13.** 2-Mercapto-5-(pyrid-3-yl)-1,3,4-thiadiazol gemäss Anspruch 1 der Formel Id

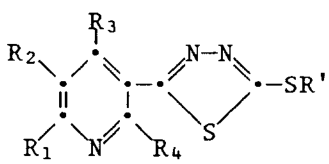

(Id),

worin R' Difluormethyl und $R_1$-$R_4$ Wasserstoff bedeuten.

**14.** 2-Mercapto-5-(pyrid-3-yl)-1,3,4-thiadiazole gemäss Anspruch 1 der Formel Id

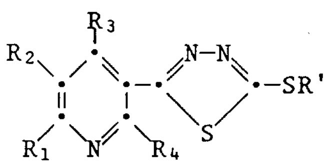

(Id),

worin R' Difluormethyl, $R_1$ Wasserstoff, $C_1$-$C_3$-Alkyl oder Chlor, $R_2$ Wasserstoff und $R_3$ und $R_4$ voneinander unabhängig Wasserstoff, Chlor oder Methoxy bedeuten.

**15.** 2-Mercapto-5-(pyrid-3-yl)-1,3,4-thiadiazole gemäss Anspruch 1 der Formel Id

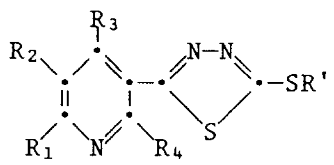

(Id),

worin R' Difluormethyl, $R_1$, $R_2$ und $R_4$ voneinander unabhängig Wasserstoff, Chlor, Brom, Methylthio oder Amino und $R_3$ Wasserstoff bedeuten.

**16.** 2-Mercapto-5-(pyrid-4-yl)-1,3,4-oxadiazol gemäss Anspruch 1 der Formel Ie

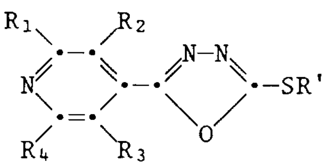

(Ie),

worin R' Difluormethyl und $R_1$-$R_4$ Wasserstoff bedeuten.

**17.** 2-Mercapto-5-(pyrid-4-yl)-1,3,4-thiadiazole gemäss Anspruch 1 der Formel If

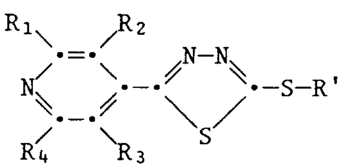

(If),

worin R' Difluormethyl, $R_1$ Wasserstoff, $C_1$-$C_3$-Alkyl, Chlor, Methoxy oder Methylmercapto, $R_4$ Wasserstoff, Methyl, Chlor, Methoxy-äthoxy, Trifluormethylmethoxy, Methylmercapto, Amino oder $C_1$-$C_3$-Monoalkylamino, und $R_2$ und $R_3$ Wasserstoff bedeuten.

**18.** 2-Difluormethylthio-5-(pyrid-4-yl)-1,3,4-thiadiazol gemäss Anspruch 17.

**19.** Verfahren zur Herstellung einer Verbindung gemäss Anspruch 1 der Formel I, dadurch gekennzeichnet, dass man in einer Kondensationsreaktion eine Verbindung der Formel II

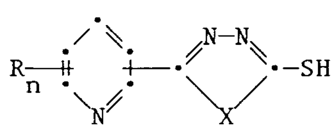

(II)

oder eine Verbindung der Formel III

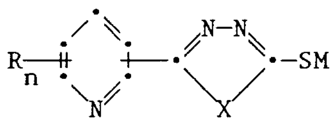

(III)

mit einer Verbindung der Formel IV

Hal-R' (IV)

in einem inerten Lösungsmittel oder Lösungsmittel-Gemisch bei erhöhter Temperatur, gegebenenfalls in Gegenwart eines Katalysators und gegebenenfalls bei erhöhtem Druck, umsetzt, wobei die Umsetzung einer Verbindung der Formel II in Gegenwart einer Base verläuft und wobei in den vorstehend genannten Formeln II, III und IV M für ein Alkalimetall oder für Ammonium steht und Hal Halogen, vorzugsweise Chlor, Brom oder Iod, bedeutet, während R, R', X und n die für die Formel I angegebenen Bedeutungen besitzen.

**20.** Schädlingsbekämpfungsmittel zur Bekämpfung oder Verhütung eines Befalls von Pflanzen durch Nematoden, dadurch gekennzeichnet, dass es als aktive Komponente mindestens eine Verbindung der Formel I gemäss Anspruch 1 enthält.

**21.** Mittel gemäss Anspruch 20, dadurch gekennzeichnet, dass es als aktive Komponente mindestens eine Verbindung der Formel I gemäss einem der Ansprüche 2 bis 18 enthält.

**22.** Mittel gemäss Anspruch 20, dadurch gekennzeichnet, dass es als aktive Komponente 2-Difluormethylthio-5-(pyrid-2-yl)-1,3,4-thiadiazol enthält.

**23.** Mittel nach einem der Ansprüche 20 bis 22, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

**24.** Mittel nach Anspruch 23, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

**25.** Verfahren zur Herstellung eines Mittels gemäss einem der Ansprüche 20 bis 24, dadurch gekennzeichnet, dass man mindestens eine Verbindung der Formel I mit geeigneten festen oder flüssigen Zusatzstoffen und Tensiden innig vermischt.

**26.** Verfahren zur Bekämpfung oder Verhütung eines Befalls von Kulturpflanzen durch Nematoden, dadurch gekennzeichnet, dass man eine Verbindung der Formel I gemäss einem der Ansprüche 1 bis 18 auf die Pflanze oder deren Standort appliziert.

**27.** Verwendung von Verbindungen der Formel I gemäss Anspruch 1 zur Bekämpfung und/oder zur präventiven Verhütung eines Befalls von Pflanzen durch Nematoden.

**28.** Verwendung gemäss Anspruch 27 von Verbindungen der Formel I gemäss einem der Ansprüche 1 bis 18.

**29.** Verwendung gemäss Anspruch 27 oder 28, dadurch gekennzeichnet, dass die Nematoden solche der Gattung Meloidogyne, Heterodera oder Globodera sind.

**Patentansprüche für folgenden Vertragsstaat : ES**

**1.** Verfahren zur Herstellung von 2-Mercapto-5-pyridyl-1,3,4-oxadiazole und 2-Mercapto-5-pyridyl-1,3,4-thiadiazole der Formel I

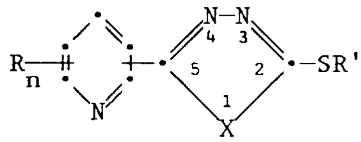

(I),

in welcher
X Sauerstoff oder Schwefel,
R' Difluormethyl und
R $C_1$-$C_3$-Alkyl, unsubstituiertes oder durch Halogen oder $C_1$-$C_3$-Alkoxy substituiertes $C_1$-$C_3$-Alkoxy; unsubstituiertes oder durch Halogen substituiertes $C_3$-$C_7$-Alkenyl; $C_3$-$C_7$-Alkinyl, $C_1$-$C_3$-Alkylthio, Halogen, Cyano, Hydroxy, unsubstituiertes oder durch $C_1$-$C_3$-Alkyl mono- oder disubstituiertes Amino; oder Aminocarbonyl bedeuten und
n für 0, 1, 2, 3 oder 4 steht, in freier Form oder in Salzform, dadurch
gekennzeichnet, dass man in einer Kondensationsreaktion eine Verbindung der Formel II

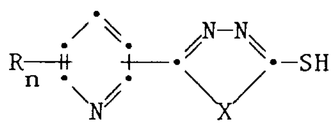

(II)

oder eine Verbindung der Formel III

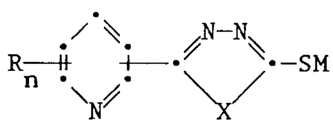

(III)

mit einer Verbindung der Formel IV

Hal-R' (IV)

in einem inerten Lösungsmittel oder Lösungsmittel-Gemisch bei erhöhter Temperatur, gegebenenfalls in Gegenwart eines Katalysators und gegebenenfalls bei erhöhtem Druck, umsetzt, wobei die Umsetzung einer Verbindung der Formel II in Gegenwart einer Base verläuft und wobei in den vorstehend genannten Formeln II, III und IV M für ein Alkalimetall oder für Ammonium steht und Hal Halogen, vorzugsweise Chlor, Brom oder Iod, bedeutet, während R, R', X und n die für die Formel I angegebenen Bedeutungen besitzen.

**2.** Verfahren gemäss Anspruch 1, dadurch gekennzeichnet, dass 2-Mercapto-5-(pyrid-2-yl)-1,3,4-oxadiazole der Formel Ia

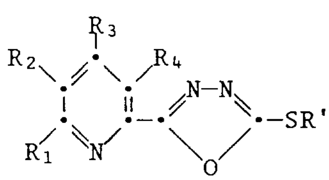

(Ia),

hergestellt werden,
worin R' Difluormethyl, die Reste $R_1$-$R_3$ Wasserstoff und $R_4$ Aminocarbonyl oder Hydroxy bedeuten.

**3.** Verfahren gemäss Anspruch 1, dadurch gekennzeichnet, dass 2-Mercapto-5-(pyrid-2-yl)-1,3,4-oxadiazol der Formel Ia

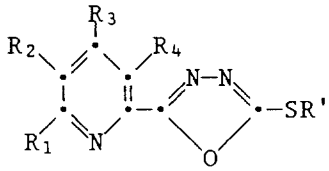

(Ia),

hergestellt wird,
worin R' Difluormethyl und $R_1$-$R_4$ Wasserstoff bedeuten.

**4.** Verfahren gemäss Anspruch 1, dadurch gekennzeichnet, dass 2-Mercapto-5-(pyrid-2-yl)-1,3,4-thiadiazole der Formel Ib

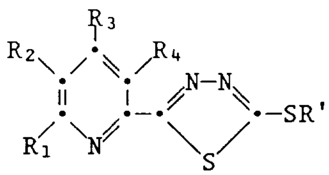

(Ib),

hergestellt werden,
worin R' Difluormethyl und $R_1$-$R_4$ voneinander unabhängig Wasserstoff, Methyl, Methoxy, Methylmercapto, Chlor, Cyano, Hydroxy, Amino oder Aminocarbonyl bedeuten.

**5.** Verfahren gemäss Anspruch 4 dadurch gekennzeichnet, dass Verbindungen der Formel Ib hergestellt werden, worin $R_1$ und $R_4$ voneinander unabhängig Wasserstoff oder Chlor und $R_2$ und $R_3$ Wasserstoff bedeuten.

**6.** Verfahren gemäss Anspruch 4 dadurch gekennzeichnet, dass Verbindungen der Formel Ib hergestellt werden, worin R' Difluormethyl, $R_1$, $R_2$ und $R_3$ Wasserstoff und $R_4$ Methyl, Methoxy, Chlor, Cyano, Aminocarbonyl, Hydroxy oder Amino bedeuten.

**7.** Verfahren gemäss Anspruch 4 dadurch gekennzeichnet, dass Verbindungen der Formel Ib hergestellt werden, worin R' Difluormethyl, einer der Reste $R_1$, $R_2$ und $R_3$ Methyl, Chlor, Methoxy oder Methylmercapto, die restlichen zwei Reste Wasserstoff und $R_4$ Wasserstoff bedeuten.

**8.** Verfahren gemäss Anspruch 4 dadurch gekennzeichnet, dass Verbindungen der Formel Ib hergestellt werden, worin R' Difluormethyl, $R_1$ und $R_4$ voneinander unabhängig Wasserstoff, Chlor, Methoxy oder Hydroxy und $R_2$ und $R_3$ Wasserstoff bedeuten.

**9.** Verfahren gemäss Anspruch 8, dadurch gekennzeichnet, dass 2-Difluormethylthio-5-(pyrid-2-yl)-1,3,4-thiadiazol hergestellt wird.

**10.** Verfahren gemäss Anspruch 1, dadurch gekennzeichnet, dass 2-Mercapto-5-(pyrid-3-yl)-1,3,4-oxadiazole der Formel Ic hergestellt werden,

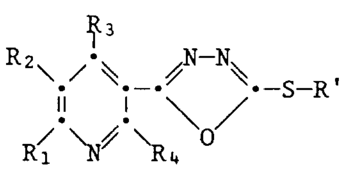

(Ic),

worin R' Difluormethyl und einer der Reste $R_1$-$R_4$ Methyl oder Methylmercapto und die restlichen drei Reste Wasserstoff bedeuten.

11. Verfahren gemäss Anspruch 1, dadurch gekennzeichnet, dass 2-Mercapto-5-(pyrid-3-yl)-1,3,4-oxadiazole der Formel Ic hergestellt werden,

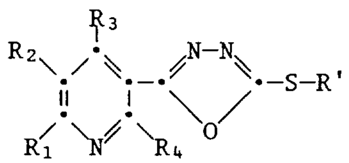

(Ic),

worin R' Difluormethyl, $R_1$ und $R_4$ voneinander unabhängig Methyl oder Chlor und $R_2$ und $R_3$ Wasserstoff bedeuten.

12. Verfahren gemäss Anspruch 1, dadurch gekennzeichnet, dass 2-Mercapto-5-(pyrid-3-yl)-1,3,4-oxadiazol der Formel Ic hergestellt wird,

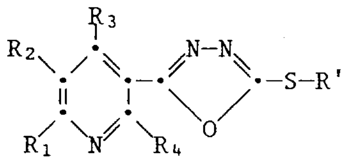

(Ic),

worin R' Difluormethyl und $R_1$-$R_4$ Wasserstoff bedeuten.

13. Verfahren gemäss Anspruch 1, dadurch gekennzeichnet, dass 2-Mercapto-5-(pyrid-3-yl)-1,3,4-thiadiazol der Formel Id hergestellt wird,

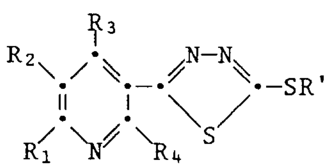

(Id),

worin R' Difluormethyl und $R_1$-$R_4$ Wasserstoff bedeuten.

14. Verfahren gemäss Anspruch 1, dadurch gekennzeichnet, dass 2-Mercapto-5-(pyrid-3-yl)-1,3,4-thiadiazole der Formel Id hergestellt werden,

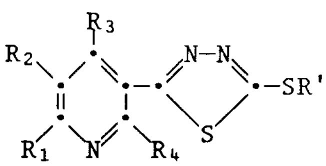

(Id),

worin R' Difluormethyl, $R_1$ Wasserstoff, $C_1$-$C_3$-Alkyl oder Chlor, $R_2$ Wasserstoff und $R_3$ und $R_4$ voneinander unabhängig Wasserstoff, Chlor oder Methoxy bedeuten.

15. Verfahren gemäss Anspruch 1, dadurch gekennzeichnet, dass 2-Mercapto-5-(pyrid-3-yl)-1,3,4-thiadiazole der Formel Id hergestellt werden,

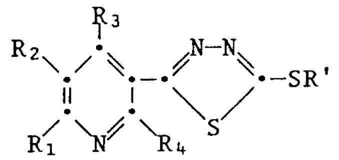

(Id),

worin R' Difluormethyl, $R_1$, $R_2$ und $R_4$ voneinander unabhängig Wasserstoff, Chlor, Brom, Methylthio oder Amino und $R_3$ Wasserstoff bedeuten.

**16.** Verfahren gemäss Anspruch 4 dadurch gekennzeichnet, dass 2-Mercapto-5-(pyrid-4-yl)-1,3,4-oxadiazol der Formel Ie hergestellt wird,

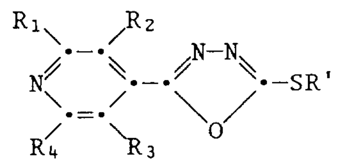

(Ie),

worin R' Difluormethyl und $R_1$-$R_4$ Wasserstoff bedeuten.

**17.** Verfahren gemäss Anspruch 4 dadurch gekennzeichnet, dass 2-Mercapto-5-(pyrid-4-yl)-1,3,4-thiadiazole der Formel If hergestellt werden,

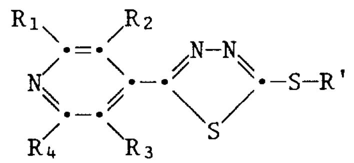

(If),

worin R' Difluormethyl, $R_1$ Wasserstoff, $C_1$-$C_3$-Alkyl, Chlor, Methoxy oder Methylmercapto, $R_4$ Wasserstoff, Methyl, Chlor, Methoxy-äthoxy, Trifluormethylmethoxy, Methylmercapto, Amino oder $C_1$-$C_3$-Monoalkylamino, und $R_2$ und $R_3$ Wasserstoff bedeuten.

**18.** Verfahren gemäss Anspruch 1, dadurch gekennzeichnet, dass 2-Difluormethylthio-5-(pyrid-4-yl)-1,3,4-thiadiazol hergestellt wird.

**19.** Schädlingsbekämpfungsmittel zur Bekämpfung oder Verhütung eines Befalls von Pflanzen durch Nematoden, dadurch gekennzeichnet, dass es als aktive Komponente mindestens eine Verbindung der Formel I gemäss Anspruch 1 enthält.

**20.** Mittel gemäss Anspruch 19, dadurch gekennzeichnet, dass es als aktive Komponente mindestens eine Verbindung der Formel I gemäss einem der Ansprüche 2 bis 18 enthält.

**21.** Mittel gemäss Anspruch 19, dadurch gekennzeichnet, dass es als aktive Komponente 2-Difluormethylthio-5-(pyrid-2-yl)-1,3,4-thiadiazol enthält.

**22.** Mittel nach einem der Ansprüche 19 bis 21, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

**23.** Mittel nach Anspruch 22, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

**24.** Verfahren zur Herstellung eines Mittels gemäss einem der Ansprüche 19 bis 23, dadurch gekennzeichnet, dass man mindestens eine Verbindung der Formel I mit geeigneten festen oder flüssigen Zusatzstoffen und Tensiden innig vermischt.

**25.** Verfahren zur Bekämpfung oder Verhütung eines Befalls von Kulturpflanzen durch Nematoden, dadurch gekennzeichnet, dass man eine Verbindung der Formel I gemäss einem der Ansprüche 1 bis 18 auf die Pflanze oder deren Standort appliziert.

**26.** Verwendung von Verbindungen der Formel I gemäss Anspruch 1 zur Bekämpfung und/oder zur präventiven Verhütung eines Befalls von Pflanzen durch Nematoden.

**27.** Verwendung gemäss Anspruch 26 von Verbindungen der Formel I gemäss einem der Ansprüche 1 bis 18.

**28.** Verwendung gemäss Anspruch 26 oder 27, dadurch gekennzeichnet, dass die Nematoden solche der Gattung Meloidogyne, Heterodera oder Globodera sind.

**Claims**

**Claims for the following Contracting States : AT, BE, CH, DE, FR, GB, GR, IT, LI, LU, NL**

**1.** A 2-mercapto-5-pyridyl-1,3,4-oxadiazole or 2-mercapto-5-pyridyl-1,3,4-thiadiazole of formula I

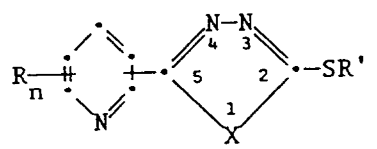

(I)

wherein

X is oxygen or sulphur,

R' is difluoromethyl,

R is $C_1$-$C_3$alkyl, unsubstituted $C_1$-$C_3$alkoxy or $C_1$-$C_3$alkoxy which is substituted by halogen or $C_1$-$C_3$alkoxy; unsubstituted or halogen-substituted $C_3$-$C_7$alkenyl; $C_3$-$C_7$alkynyl, $C_1$-$C_3$alkylthio, halogen, cyano, hydroxy, unsubstituted amino or amino which is substituted by one or two $C_1$-$C_3$alkyl groups; or is aminocarbonyl; and

n is 0, 1, 2, 3 or 4,

in free form or in salt form.

**2.** A 2-mercapto-5-(pyrid-2-yl)-1,3,4-oxadiazole according to claim 1 of formula Ia

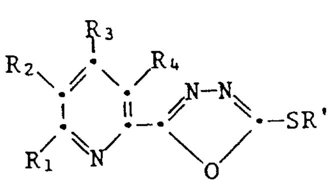

(Ia)

wherein R' is difluoromethyl, the radicals $R_1$, $R_2$ and $R_3$ are hydrogen and $R_4$ is aminocarbonyl or hydroxy.

**3.** A 2-mercapto-5-(pyrid-2-yl)-1,3,4-oxadiazole according to claim 1 of formula Ia

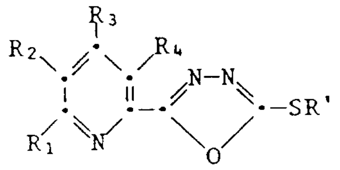

(Ia)

wherein R' is difluoromethyl and $R_1$, $R_2$, $R_3$ and $R_4$ are hydrogen.

**4.** A 2-mercapto-5-(pyrid-2-yl)-1,3,4-thiadiazole according to claim 1 of formula Ib

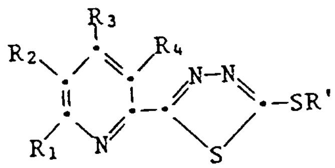

(Ib)

wherein R' is difluoromethyl and $R_1$, $R_2$, $R_3$ and $R_4$ are each independently hydrogen, methyl, methoxy, methylmercapto, chlorine, cyano, hydroxy, amino or aminocarbonyl.

**5.** A compound according to claim 4 of formula Ib, wherein $R_1$ and $R_4$ are each independently of the other hydrogen or chlorine and $R_2$ and $R_3$ are hydrogen.

**6.** A compound according to claim 4 of formula Ib, wherein R' is difluoromethyl, $R_1$, $R_2$ and $R_3$ are hydrogen and $R_4$ is methyl, methoxy, chlorine, cyano, aminocarbonyl, hydroxy or amino.

**7.** A compound according to claim 4 of formula Ib, wherein R' is difluoromethyl, one of the radicals $R_1$, $R_2$ and $R_3$ is methyl, chlorine, methoxy or methylmercapto and the other two radicals are hydrogen and $R_4$ is hydrogen.

**8.** A compound according to claim 4 of formula Ib, wherein R' is difluoromethyl, $R_1$ and $R_4$ are each independently of the other hydrogen, chlorine, methoxy or hydroxy, and $R_2$ and $R_3$ are hydrogen.

**9.** 2-difluoromethylthio-5-(pyrid-2-yl)-1,3,4-thiadiazole according to claim 8.

**10.** A 2-mercapto-5-(pyrid-3-yl)-1,3,4-oxadiazole according to claim 1 of formula Ic

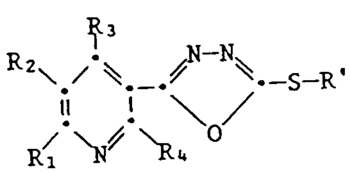

(Ic)

wherein R' is difluoromethyl and one of the radicals $R_1$, $R_2$, $R_3$ and $R_4$ is methyl or methylmercapto and the other three radicals are hydrogen.

**11.** A 2-mercapto-5-(pyrid-3-yl)-1,3,4-oxadiazole according to claim 1 of formula Ic

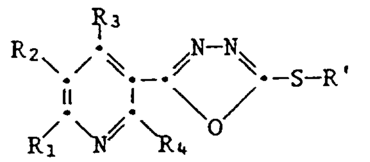

(Ic)

wherein R' is difluoromethyl, $R_1$ and $R_4$ are each independently of the other methyl or chlorine and $R_2$ and $R_3$ are hydrogen.

**12.** A 2-mercapto-5-(pyrid-3-yl)-1,3,4-oxadiazole according to claim 1 of formula Ic

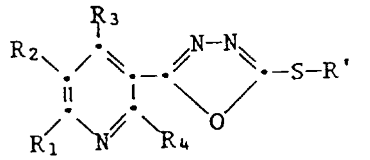

(Ic)

wherein R' is difluoromethyl and $R_1$, $R_2$, $R_3$ and $R_4$ are hydrogen.

**13.** A 2-mercapto-5-(pyrid-3-yl)-1,3,4-thiadiazole according to claim 1 of formula Id

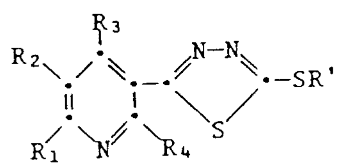

(Id)

wherein R' is difluoromethyl and $R_1$, $R_2$, $R_3$ and $R_4$ are hydrogen.

**14.** A 2-mercapto-5-(pyrid-3-yl)-1,3,4-thiadiazole according to claim 1 of formula Id

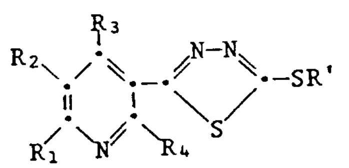

(Id)

wherein R' is difluoromethyl, $R_1$ is hydrogen, $C_1$-$C_3$alkyl or chlorine, $R_2$ is hydrogen and $R_3$ and $R_4$ are each independently of the other hydrogen, chlorine or methoxy.

**15.** A 2-mercapto-5-(pyrid-3-yl)-1,3,4-thiadiazole according to claim 1 of formula Id

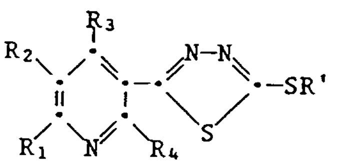

(Id)

wherein R' is difluoromethyl, $R_1$, $R_2$ and $R_4$ are each independently hydrogen, chlorine, bromine, methylthio or amino, and $R_3$ is hydrogen.

**16.** A 2-mercapto-5-(pyrid-4-yl)-1,3,4-oxadiazole according to claim 1 of formula Ie

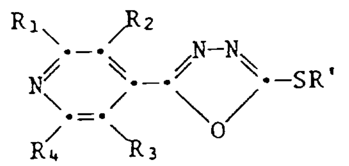

(Ie)

wherein R' is difluoromethyl and $R_1$, $R_2$, $R_3$ and $R_4$ are hydrogen.

**17.** A 2-mercapto-5-(pyrid-4-yl)-1,3,4-thiadiazole according to claim 1 of formula If

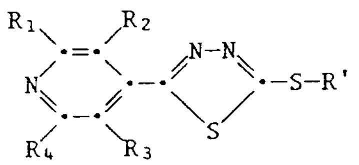

(If)

wherein R' is difluoromethyl, $R_1$ is hydrogen, $C_1$-$C_3$alkyl, chlorine, methoxy or methylmercapto, $R_4$ is hydrogen, methyl, chlorine, methoxyethoxy, trifluoromethylmethoxy, methylmercapto, amino or $C_1$-$C_3$monoalkylamino and $R_2$ and $R_3$ are hydrogen.

**18.** 2-difluoromethylthio-5-(pyrid-4-yl)-1,3,4-thiadiazole according to claim 17.

**19.** A process for the preparation of a compound according to claim 1 of formula I, which comprises condensing a compound of formula II

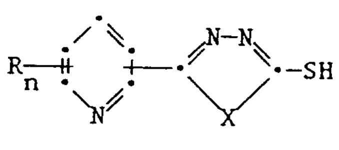

(II)

or a compound of formula III

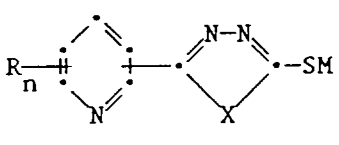

(III),

with a compound of formula IV

Hal-R' (IV)

in an inert solvent or mixture of solvents at elevated temperature, in the absence or presence of a catalyst and under normal or elevated pressure, the reaction of a compound of formula II being carried out in the presence of a base and in which formulae II, III and IV above M is an alkali metal or ammonium and Hal is halogen, preferably chlorine, bromine or iodine, while R, R', X and n are as defined for formula I.

**20.** A pesticidal composition for controlling nematodes or for protecting plants from attack by nematodes, which composition comprises as active component at least one compound of formula I as claimed in claim 1.

**21.** A composition according to claim 20, which comprises as active component at least one compound of formula I as claimed in any one of claims 2 to 18.

**22.** A composition according to claim 20, which comprises 2-difluoromethylthio-5-(pyrid-2-yl)-1,3,4-thiadiazole as active component.

**23.** A composition according to any one of claims 20 to 22, which comprises 0.1 to 99 % of a compound of formula I, 99.9 to 1 % of a solid or liquid adjuvant and 0 to 25 % of a surfactant.

**24.** A composition according to claim 23, which comprises 0.1 to 95 % of a compound of formula I, 99.8 to 5 % of a solid or liquid adjuvant and 0.1 to 25 % of a surfactant.

**25.** A process for the preparation of a composition as claimed in any one of claims 20 to 24, which comprises homogeneously mixing at least one compound of formula I with suitable solid or liquid adjuvants and surfactants.

**26.** A method of controlling nematodes or of protecting cultivated plants from attack by nematodes, which method comprises applying to the plant or to the locus thereof a compound of formula I as claimed in any one of claims 1 to 18.

**27.** The use of a compound of formula I as claimed in claim 1 for controlling nematodes and/or for protecting plants from attack by nematodes.

**28.** The use according to claim 27 of a compound of formula I as claimed in any one of claims 1 to 18.

**29.** The use according to claim 27 or claim 28, wherein the nematodes are of the genus Meloidogyne, Heterodera or Globodera.

**Claims for the following Contracting State : ES**

**1.** A process for the preparation of a 2-mercapto-5-pyridyl-1,3,4-oxadiazole or 2-mercapto-5-pyridyl-1,3,4-thiadiazole of formula I

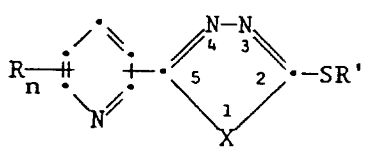

(I)

wherein

X is oxygen or sulphur,

R' is difluoromethyl,

R is $C_1$-$C_3$alkyl, unsubstituted $C_1$-$C_3$alkoxy or $C_1$-$C_3$alkoxy which is substituted by halogen or $C_1$-$C_3$alkoxy; unsubstituted or halogen-substituted $C_3$-$C_7$alkenyl; $C_3$-$C_7$alkynyl, $C_1$-$C_3$alkylthio, halogen, cyano, hydroxy, unsubstituted amino or amino which is substituted by one or two $C_1$-$C_3$alkyl groups; or is aminocarbonyl; and

n is 0, 1, 2, 3 or 4, in free form or in salt form,

which comprises condensing a compound of formula II

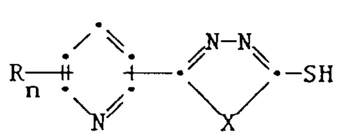

(II)

or a compound of formula III

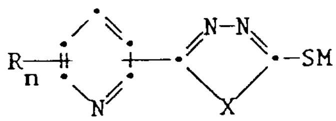

(III),

with a compound of formula IV

Hal-R' (IV)

in an inert solvent or mixture of solvents at elevated temperature, in the absence or presence of a catalyst and under normal or elevated pressure, the reaction of a compound of formula II being carried out in the presence of a base and in which formulae II, III and IV above M is an alkali metal or ammonium and Hal is halogen, preferably chlorine, bromine or iodine, while R, R', X and n are as defined for formula I.

**2.** A process according to claim 1, which comprises preparing a 2-mercapto-5-(pyrid-2-yl)-1,3,4-oxadiazole of formula Ia

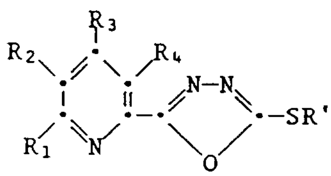

(Ia)

wherein R' is difluoromethyl, the radicals $R_1$, $R_2$ and $R_3$ are hydrogen and $R_4$ is aminocarbonyl or hydroxy.

**3.** A process according to claim 1, which comprises preparing a 2-mercapto-5-(pyrid-2-yl)-1,3,4-oxadiazole of formula Ia

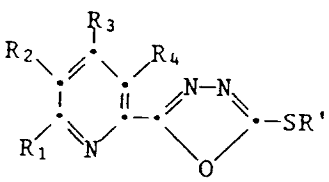

(Ia)

wherein R' is difluoromethyl and $R_1$, $R_2$, $R_3$ and $R_4$ are hydrogen.

**4.** A process according to claim 1, which comprises preparing a 2-mercapto-5-(pyrid-2-yl)-1,3,4-thiadiazole of formula Ib

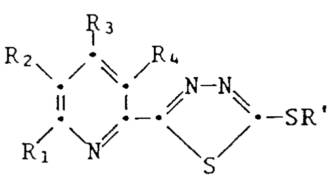

(Ib)

wherein R' is difluoromethyl and $R_1$, $R_2$, $R_3$ and $R_4$ are each independently hydrogen, methyl, methoxy, methylmercapto, chlorine, cyano, hydroxy, amino or aminocarbonyl.

**5.** A process according to claim 4, which comprises preparing a compound of formula Ib wherein $R_1$ and $R_4$ are each independently of the other hydrogen or chlorine and $R_2$ and $R_3$ are hydrogen.

**6.** A process according to claim 4, which comprises preparing a compound of formula Ib wherein R' is difluoromethyl, $R_1$, $R_2$ and $R_3$ are hydrogen and $R_4$ is methyl, methoxy, chlorine, cyano, aminocarbonyl, hydroxy or amino.

**7.** A process according to claim 4, which comprises preparing a compound of formula Ib wherein R' is difluoromethyl, one of the radicals $R_1$, $R_2$ and $R_3$ is methyl, chlorine, methoxy or methylmercapto and the other two radicals are hydrogen and $R_4$ is hydrogen.

**8.** A process according to claim 4, which comprises preparing a compound of formula Ib wherein R' is difluoromethyl, $R_1$ and $R_4$ are each independently of the other hydrogen, chlorine, methoxy or hydroxy, and $R_2$ and $R_3$ are hydrogen.

**9.** A process according to claim 8, which comprises preparing 2-difluoromethylthio-5-(pyrid-2-yl)-1,3,4-thiadiazole.

**10.** A process according to claim 1, which comprises preparing a 2-mercapto-5-(pyrid-3-yl)-1,3,4-oxadiazole of formula Ic

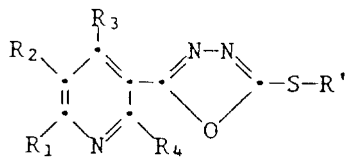

(Ic)

wherein R' is difluoromethyl and one of the radicals $R_1$, $R_2$, $R_3$ and $R_4$ is methyl or methylmercapto and the other three radicals are hydrogen.

**11.** A process according to claim 1, which comprises preparing a 2-mercapto-5-(pyrid-3-yl)-1,3,4-oxadiazole of formula Ic

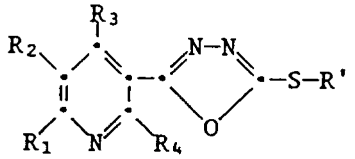

(Ic)

wherein R' is difluoromethyl, $R_1$ and $R_4$ are each independently of the other methyl or chlorine and $R_2$ and $R_3$ are hydrogen.

**12.** A process according to claim 1, which comprises preparing a 2-mercapto-5-(pyrid-3-yl)-1,3,4-oxadiazole of formula Ic

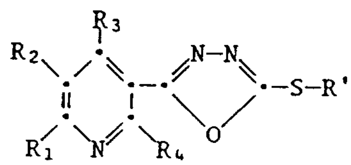

(Ic)

wherein R' is difluoromethyl and $R_1$, $R_2$, $R_3$ and $R_4$ are hydrogen.

**13.** A process according to claim 1, which comprises preparing a 2-mercapto-5-(pyrid-3-yl)-1,3,4-thiadiazole of formula Id

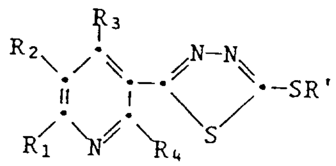

(Id)

wherein R' is difluoromethyl and $R_1$, $R_2$, $R_3$ and $R_4$ are hydrogen.

**14.** A process according to claim 1, which comprises preparing a 2-mercapto-5-(pyrid-3-yl)-1,3,4-thiadiazole of formula Id

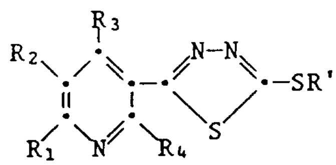

(Id)

wherein R' is difluoromethyl, $R_1$ is hydrogen, $C_1$-$C_3$alkyl or chlorine, $R_2$ is hydrogen and $R_3$ and $R_4$ are each independently of the other hydrogen, chlorine or methoxy.

**15.** A process according to claim 1, which comprises preparing a 2-mercapto-5-(pyrid-3-yl)-1,3,4-thiadiazole of formula Id

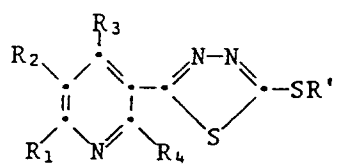

(Id)

wherein R' is difluoromethyl, $R_1$, $R_2$ and $R_4$ are each independently hydrogen, chlorine, bromine, methylthio or amino, and $R_3$ is hydrogen.

**16.** A process according to claim 4, which comprises preparing a 2-mercapto-5-(pyrid-4-yl)-1,3,4-oxadiazole of formula Ie

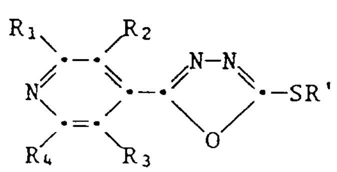

(Ie)

wherein R' is difluoromethyl and $R_1$, $R_2$, $R_3$ and $R_4$ are hydrogen.

**17.** A process according to claim 4, which comprises preparing a 2-mercapto-5-(pyrid-4-yl)-1,3,4-thiadiazole of formula If

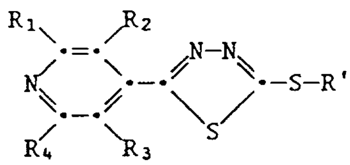

(If)

wherein R' is difluoromethyl, $R_1$ is hydrogen, $C_1$-$C_3$alkyl, chlorine, methoxy or methylmercapto, $R_4$ is hydrogen, methyl, chlorine, methoxyethoxy, trifluoromethylmethoxy, methylmercapto, amino or $C_1$-$C_3$monoalkylamino and $R_2$ and $R_3$ are hydrogen.

**18.** A process according to claim 1, which comprises preparing 2-difluoromethylthio-5-(pyrid-4-yl)-1,3,4-thiadiazole.

**19.** A pesticidal composition for controlling nematodes or for protecting plants from attack by nematodes, which composition comprises as active component at least one compound of formula I as claimed in claim 1.

**20.** A composition according to claim 19, which comprises as active component at least one compound of formula I as claimed in any one of claims 2 to 18.

**21.** A composition according to claim 19, which comprises 2-difluoromethylthio-5-(pyrid-2-yl)-1,3,4-thiadiazole as active component.

**22.** A composition according to any one of claims 19 to 21, which comprises 0.1 to 99 % of a compound of formula I, 99.9 to 1 % of a solid or liquid adjuvant and 0 to 25 % of a surfactant.

**23.** A composition according to claim 22, which comprises 0.1 to 95 % of a compound of formula I, 99.8 to 5 % of a solid or liquid adjuvant and 0.1 to 25 % of a surfactant.

**24.** A process for the preparation of a composition as claimed in any one of claims 19 to 23, which comprises homogeneously mixing at least one compound of formula I with suitable solid or liquid adjuvants and surfactants.

**25.** A method of controlling nematodes or of protecting cultivated plants from attack by nematodes, which method comprises applying to the plant or to the locus thereof a compound of formula I as claimed in any one of claims 1 to 18.

**26.** The use of a compound of formula I as claimed in claim 1 for controlling nematodes and/or for protecting plants from attack by nematodes.

**27.** The use according to claim 26 of a compound of formula I as claimed in any one of claims 1 to 18.

**28.** The use according to claim 26 or claim 27, wherein the nematodes are of the genus Meloidogyne, Heterodera or Globodera.

**Revendications**

**Revendications pour les Etats contractants suivants : AT, BE, CH, DE, FR, GB, GR, IT, LI, LU, NL**

**1.** 2-mercapto-5-pyridyl-1,3,4-oxadiazoles et 2-mercapto-5-pyridyl-1,3,4-thiadiazoles de formule I

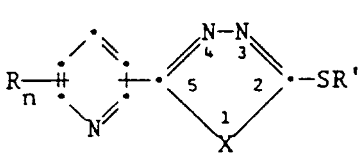

(I),

dans laquelle

X représente l'oxygène ou le soufre,

R' représente un groupe difluorométhyle et

R représente un groupe alkyle en C1-C3, un groupe alcoxy en C1-C3 non substitué ou substitué par un halogène ou par un groupe alcoxy en C1-C3; un groupe alcényle en C3-C7 non substitué ou substitué par un halogène; un groupe alcynyle en C3-C7, alkylthio en C1-C3, un halogène, un groupe cyano, hydroxy, un groupe amino non substitué ou mono- ou di-substitué par des groupes alkyle en C1-C3; ou un groupe aminocarbonyle et

n est égal à 0, 1, 2, 3 ou 4, à l'état libre ou à l'état de sels.

**2.** 2-mercapto-5-(pyrido-2-yl)-1,3,4-oxadiazoles selon revendication 1, de formule Ia

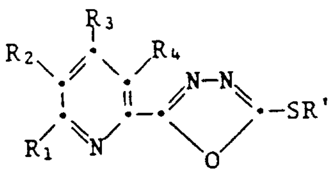

(Ia),

dans laquelle R' représente un groupe difluorométhyle, les symboles $R_1$ à $R_3$ l'hydrogène et $R_4$ un groupe aminocarbonyle ou hydroxy.

**3.** 2-mercapto-5-(pyrido-2-yl)-1,3,4-oxadiazole selon revendication 1, de formule Ia

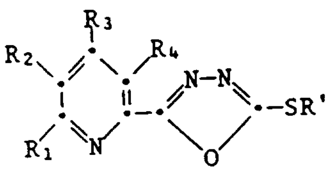

(Ia),

dans laquelle R' représente un groupe difluorométhyle et $R_1$ à $R_4$ l'hydrogène.

**4.** 2-mercapto-5-(pyrido-2-yl)-1,3,4-thiadiazoles selon revendication 1, de formule Ib

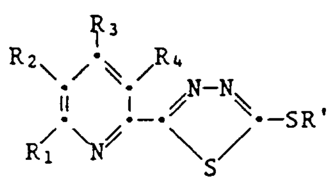

(Ib),

dans laquelle R' représente un groupe difluorométhyle et les symboles $R_1$ à $R_4$ représentent chacun, indépendamment les uns des autres, l'hydrogène, un groupe méthyle, méthoxy, méthylmercapto, le chlore, un groupe cyano, hydroxy, amino ou aminocarbonyle.

**5.** Composés selon revendication 4, de formule Ib dans laquelle $R_1$ et $R_4$ représentent chacun, indépendamment l'un de l'autre, l'hydrogène ou le chlore et $R_2$ et $R_3$ l'hydrogène.

**6.** Composés selon revendication 4, de formule Ib dans laquelle R' représente un groupe difluorométhyle, $R_1$, $R_2$ et $R_3$ l'hydrogène et $R_4$ un groupe méthyle, méthoxy, le chlore, un groupe cyano, aminocarbonyle,hydroxy ou amino.

**7.** Composés selon revendication 4, de formule Ib dans laquelle R' représente un groupe difluorométhyle, l'un des symboles $R_1$, $R_2$ et $R_3$ représente un groupe méthyle, le chlore, un groupe méthoxy ou

méthylmercapto et les deux autres l'hydrogène, et $R_4$ représente l'hydrogène.

**8.** Composés selon revendication 4, de formule Ib dans laquelle R' représente un groupe difluorométhyle, $R_1$ et $R_4$ représentent chacun, indépendamment l'un de l'autre, l'hydrogène, le chlore, un groupe méthoxy ou hydroxy et $R_2$ et $R_3$ représentent l'hydrogène.

**9.** Le 2-difluorométhylthio-5-(pyrido-2-yl)-1,3,4-thiadiazole selon revendication 8.

**10.** Les 2-mercapto-5-(pyrido-3-yl)-1,3,4-oxadiazoles selon revendication 1, de formule Ic

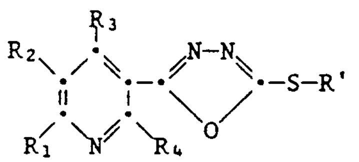

(Ic),

dans laquelle R' représente un groupe difluorométhyle et l'un des symboles $R_1$ à $R_4$ un groupe méthyle ou méthylmercapto et les trois autres l'hydrogène.

**11.** Les 2-mercapto-5-(pyrido-3-yl)-1,3,4-oxadiazoles selon revendication 1, de formule Ic

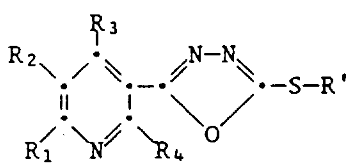

(Ic),

dans laquelle R' représente un groupe difluorométhyle, $R_1$ et $R_4$ représentent chacun, indépendamment l'un de l'autre, un groupe méthyle ou le chlore et $R_2$ et $R_3$ l'hydrogène.

**12.** Le 2-mercapto-5-(pyrido-3-yl)-1,3,4-oxadiazole selon revendication 1, de formule Ic

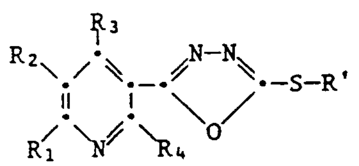

(Ic),

dans laquelle R' représente un groupe difluorométhyle et $R_1$ à $R_4$ l'hydrogène.

**13.** Le 2-mercapto-5-(pyrido-3-yl)-1,3,4-thiadiazole selon revendication 1, de formule Id

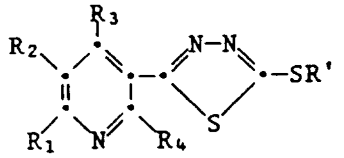

(Id),

dans laquelle R' représente un groupe difluorométhyle et $R_1$ à $R_4$ l'hydrogène.

**14.** Les 2-mercapto-5-(pyrido-3-yl)-1,3,4-thiadiazoles selon revendication 1, de formule Id

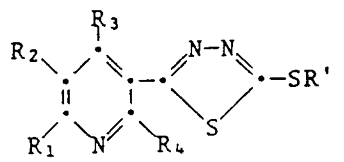

(Id),

dans laquelle R' représente un groupe difluorométhyle, $R_1$ l'hydrogène, un groupe alkyle en C1-C3 ou le chlore, $R_2$ l'hydrogène et $R_3$ et $R_4$, indépendamment l'un de l'autre, l'hydrogène, le chlore ou un groupe méthoxy.

**15.** Les 2-mercapto-5-(pyrido-3-yl)-1,3,4-thiadiazoles selon revendication 1, de formule Id

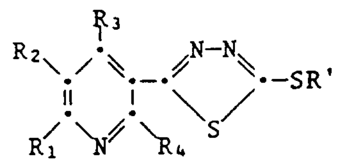

(Id),

dans laquelle R' représente un groupe difluorométhyle, $R_1$, $R_2$ et $R_4$, indépendamment les uns des autres, l'hydrogène, le chlore, le brome, un groupe méthylthio ou amino et $R_3$ l'hydrogène.

**16.** Le 2-mercapto-5-(pyrido-4-yl)-1,3,4-oxadiazole selon revendication 1, de formule Ie

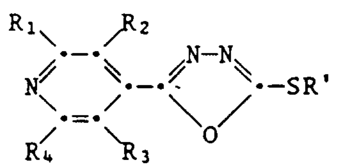

(Ie),

dans laquelle R' représente un groupe difluorométhyle et $R_1$ à $R_4$ l'hydrogène.

**17.** Les 2-mercapto-5-(pyrido-4-yl)-1,3,4-thiadiazoles selon revendication 1, de formule If

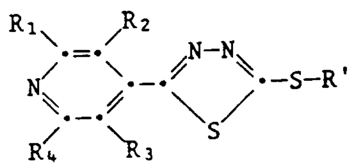

(If),

dans laquelle R' représente un groupe difluorométhyle, $R_1$ l'hydrogène, un groupe alkyle en C1-C3, le chlore, un groupe méthoxy ou méthylmercapto, $R_4$ l'hydrogène, un groupe méthyle, le chlore, un groupe méthoxyéthoxy, trifluorométhylméthoxy, méthylmercapto, amino ou monoalkylamino en C1-C3, et $R_2$ et $R_3$ l'hydrogène.

**18.** Le 2-difluorométhylthio-5-(pyrido-4-yl)-1,3,4-thiadiazole selon revendication 17.

**19.** Procédé de préparation d'un composé de formule I selon revendication 1 caractérisé en ce que, dans une réaction de condensation, on fait réagir un composé de formule II

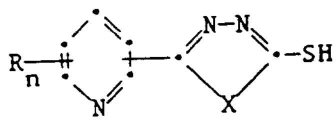

(II)

ou un composé de formule III

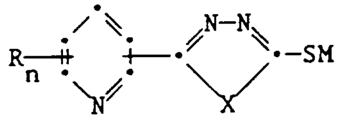

(III)

avec un composé de formule IV

Hal-R' (IV)

dans un solvant ou mélange solvant inerte, à chaud, éventuellement en présence d'un catalyseur et éventuellement sous pression, en présence d'une base dans le cas où on utilise un composé de formule II, les symboles des formules II, III et IV ci-dessus ayant les significations suivantes : M représente un métal alcalin ou l'ammonium et Hal représente un halogène, de préférence le chlore, le brome ou l'iode, et R, R', X et n ont les significations indiquées en référence à la formule I.

**20.** Produit parasiticide prévu pour combattre ou prévenir une attaque de végétaux par des nématodes, caractérisé en ce qu'il contient au moins un composant actif consistant en un composé de formule I de la revendication 1.

**21.** Produit selon revendication 20, caractérisé en ce qu'il contient au moins un composant actif consistant en un composé de formule I selon l'une des revendications 2 à 18.

**22.** Produit selon revendication 20, caractérisé en ce qu'il contient en tant que composant actif le 2-difluorométhylthio-5-(pyrido-2-yl)-1,3,4-thiadiazole.

**23.** Produit selon l'une des revendications 20 à 22, caractérisé en ce qu'il contient de 0,1 à 99 % d'une substance active de formule I, de 99,9 à 1 % d'un additif solide ou liquide et de 0 à 25 % d'un agent tensio-actif.

**24.** Produit selon revendication 23, caractérisé en ce qu'il contient de 0,1 à 95 % d'une substance active de formule I, de 99,8 à 5 % d'un additif solide ou liquide et de 0,1 à 25 % d'un agent tensio-actif.

**25.** Procédé de préparation d'un produit selon l'une des revendications 20 à 24, caractérisé en ce que l'on mélange au moins un composé de formule I avec des additifs solides ou liquides appropriés et des agents tensio-actifs.

**26.** Procédé pour combattre ou prévenir une attaque de végétaux cultivés par des nématodes, caractérisé en ce que l'on applique sur les végétaux ou leur habitat un composé de formule I selon l'une des revendications 1 à 18.

**27.** Utilisation des composés de formule I de la revendication 1 pour le traitement et/ou la prévention d'une attaque de végétaux par des nématodes.

**28.** Utilisation selon revendication 27 de composés de formule I selon l'une des revendications 1 à 18.

**29.** Utilisation selon revendication 27 ou 28, caractérisée en ce que les nématodes appartiennent au genre Meloidogyne, Heterodera ou Globodera.

**Revendications pour l'Etat contractant suivant : ES**

1. Procédé de préparation des 2-mercapto-5-pyridyl-1,3,4-oxadiazoles et 2-mercapto-5-pyridyl-1,3,4-thiadiazoles de formule I

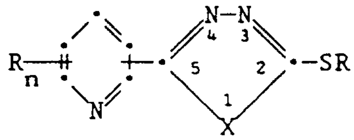

(I),

dans laquelle

X représente l'oxygène ou le soufre,

R' représente un groupe difluorométhyle et

R représente un groupe alkyle en C1-C3, un groupe alcoxy en C1-C3 non substitué ou substitué par un halogène ou par un groupe alcoxy en C1-C3; un groupe alcényle en C3-C7 non substitué ou substitué par un halogène; un groupe alcynyle en C3-C7, alkylthio en C1-C3, un halogène, un groupe cyano, hydroxy, un groupe amino non substitué ou mono- ou di-substitué par des groupes alkyle en C1-C3; ou un groupe aminocarbonyle et

n est égal à 0, 1, 2, 3 ou 4, à l'état libre ou à l'état de sels,

caractérisé en ce que, dans une réaction de condensation, on fait réagir un composé de formule II

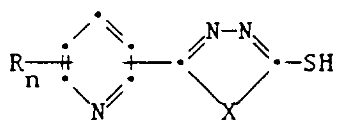

(II)

ou un composé de formule III

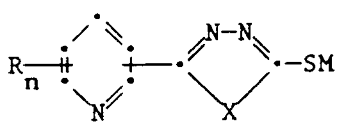

(III)

avec un composé de formule IV

Hal-R' (IV)

dans un solvant ou mélange solvant inerte, à chaud, éventuellement en présence d'un catalyseur et éventuellement sous pression, en présence d'une base dans le cas où on utilise un composé de formule II, les symboles des formules II, III et IV ci-dessus ayant les significations suivantes : M représente un métal alcalin ou l'ammonium et Hal représente un halogène, de préférence le chlore, le brome ou l'iode, et R, R', X et n ont les significations indiquées en référence à la formule I.

2. Procédé selon revendication 1, caractérisé en ce que l'on prépare les 2-mercapto-5-(pyrido-2-yl)-1,3,4-oxadiazoles de formule Ia

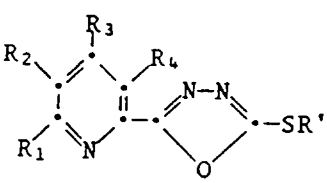

(Ia),

dans laquelle R' représente un groupe difluorométhyle, les symboles $R_1$ à $R_3$ l'hydrogène et $R_4$ un groupe aminocarbonyle ou hydroxy.

**3.** Procédé selon revendication 1, caractérisé en ce que l'on prépare le 2-mercapto-5-(pyrido-2-yl)-1,3,4-oxadiazole de formule Ia

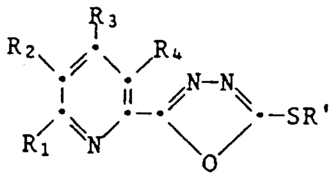

(Ia),

dans laquelle R' représente un groupe difluorométhyle et $R_1$ à $R_4$ l'hydrogène.

**4.** Procédé selon revendication 1, caractérisé en ce que l'on prépare les 2-mercapto-5-(pyrido-2-yl)-1,3,4-thiadiazoles de formule Ib

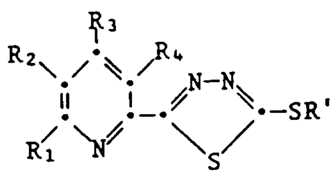

(Ib),

dans laquelle R' représente un groupe difluorométhyle et $R_1$ à $R_4$, indépendamment les uns des autres, l'hydrogène, un groupe méthyle, méthoxy, méthylmercapto, le chlore, un groupe cyano, hydroxy, amino ou aminocarbonyle.

**5.** Procédé selon revendication 4, caractérisé en ce que l'on prépare des composés de formule Ib dans laquelle $R_1$ et $R_4$ représentent, indépendamment l'un de l'autre, l'hydrogène ou le chlore et $R_2$ et $R_3$ l'hydrogène.

**6.** Procédé selon revendication 4, caractérisé en ce que l'on prépare des composés de formule Ib dans laquelle R' représente un groupe difluorométhyle, $R_1$, $R_2$ et $R_3$ l'hydrogène et $R_4$ un groupe méthyle, méthoxy, le chlore, un groupe cyano, aminocarbonyle, hydroxy ou amino.

**7.** Procédé selon revendication 4, caractérisé en ce que l'on prépare des composés de formule Ib dans laquelle R' représente un groupe difluorométhyle, l'un des symboles $R_1$, $R_2$ et $R_3$ représente un groupe méthyle, le chlore, un groupe méthoxy ou méthylmercapto et les deux autres l'hydrogène, et $R_4$ l'hydrogène.

**8.** Procédé selon revendication 4, caractérisé en ce que l'on prépare des composés de formule Ib dans laquelle R' représente un groupe difluorométhyle, $R_1$ et $R_4$ représentent chacun, indépendamment l'un de l'autre, l'hydrogène, le chlore, un groupe méthoxy ou hydroxy et $R_2$ et $R_3$ l'hydrogène.

**9.** Procédé selon revendication 8, caractérisé en ce que l'on prépare le 2-difluorométhylthio-5-(pyrido-2-yl)-1,3,4-thiadiazole.

**10.** Procédé selon revendication 1, caractérisé en ce que l'on prépare les 2-mercapto-5-(pyrido-3-yl)-1,3,4-oxadiazoles de formule Ic

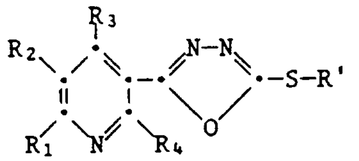

(Ic),

dans laquelle R' représente un groupe difluorométhyle, l'un des symboles $R_1$ à $R_4$ un groupe méthyle ou méthylmercapto et les trois autres l'hydrogène.

**11.** Procédé selon revendication 1, caractérisé en ce que l'on prépare les 2-mercapto-5-(pyrido-3-yl)-1,3,4-oxadiazoles de formule Ic

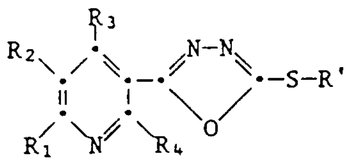

(Ic),

dans laquelle R' représente un groupe difluorométhyle, $R_1$ et $R_4$, indépendamment l'un de l'autre, un groupe méthyle ou le chlore et $R_2$ et $R_3$ l'hydrogène.

**12.** Procédé selon revendication 1, caractérisé en ce que l'on prépare le 2-mercapto-5-(pyrido-3-yl)-1,3,4-oxadiazole de formule Ic

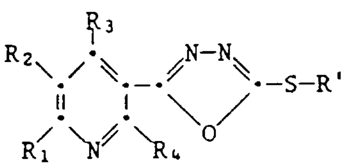

(Ic),

dans laquelle R' représente un groupe difluorométhyle et $R_1$ à $R_4$, l'hydrogène.

**13.** Procédé selon revendication 1, caractérisé en ce que l'on prépare le 2-mercapto-5-(pyrido-3-yl)-1,3,4-thiadiazolede formule Id

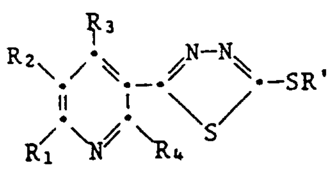

(Id),

dans laquelle R' représente un groupe difluorométhyle et $R_1$ à $R_4$, l'hydrogène.

**14.** Procédé selon revendication 1, caractérisé en ce que l'on prépare les 2-mercapto-5-(pyrido-3-yl)-1,3,4-thiadiazoles de formule Id

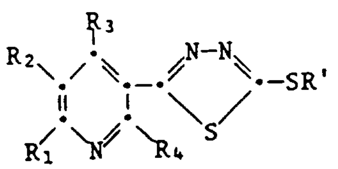

(Id),

dans laquelle R' représente un groupe difluorométhyle, $R_1$ l'hydrogène, un groupe alkyle en C1-C3 ou le chlore, $R_2$ l'hydrogène et $R_3$ et $R_4$ représentent chacun, indépendamment l'un de l'autre, l'hydrogène, le chlore ou un groupe méthoxy.

**15.** Procédé selon revendication 1, caractérisé en ce que l'on prépare les 2-mercapto-5-(pyrido-3-yl)-1,3,4-thiadiazoles de formule Id

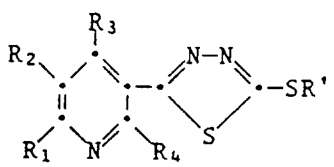

(Id),

dans laquelle R' représente un groupe difluorométhyle, $R_1$, $R_2$ et $R_4$ représentent chacun, indépendamment les uns des autres, l'hydrogène, le chlore, le brome, un groupe méthylthio ou amino et $R_3$ l'hydrogène.

**16.** Procédé selon revendication 4, caractérisé en ce que l'on prépare le 2-mercapto-5-(pyrido-4-yl)-1,3,4-oxadiazole de formule Ie

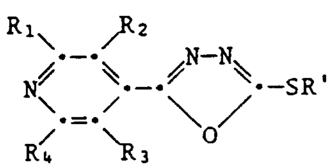

(Ie),

dans laquelle R' représente un groupe difluorométhyle et $R_1$ à $R_4$ l'hydrogène.

**17.** Procédé selon revendication 4, caractérisé en ce que l'on prépare les 2-mercapto-5-(pyrido-4-yl)-1,3,4-thiadiazoles de formule If

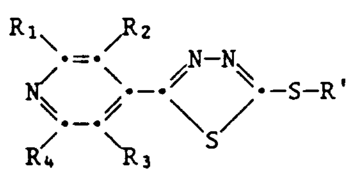

(If),

dans laquelle R' représente un groupe difluorométhyle, $R_1$ l'hydrogène, un groupe alkyle en C1-C3, le chlore, un groupe méthoxy ou méthylmercapto, $R_4$ l'hydrogène, un groupe méthyle, le chlore, un groupe méthoxyéthoxy, trifluorométhylméthoxy, méthylmercapto, amino ou monoalkylamino en C1-C3 et $R_2$ et $R_3$ l'hydrogène.

**18.** Procédé selon revendication 1, caractérise en ce que l'on prépare le 2-difluorométhylthio-5-(pyrido-4-yl)-1,3,4-thiadiazole.

**19.** Produit parasiticide prévu pour combattre ou prévenir une attaque de végétaux par des nématodes, caractérisé en ce qu'il contient au moins un composant actif consistant en un composé de formule I de la revendication 1.

**20.** Produit selon revendication 19, caractérisé en ce qu'il contient au moins un composant actif consistant en un composé de formule I selon l'une des revendications 2 à 18.

**21.** Produit selon revendication 19, caractérisé en ce qu'il contient en tant que composant actif le 2-difluorométhylthio-5-(pyrido-2-yl)-1,3,4-thiadiazole.

**22.** Produit selon l'une des revendications 19 à 21, caractérisé en ce qu'il contient de 0,1 à 99 % d'une substance active de formule I, de 99,9 à 1 % d'un additif solide ou liquide et de 0 à 25 % d'un agent tensio-actif.

**23.** Produit selon revendication 22, caractérisé en ce qu'il contient de 0,1 à 95 % d'une substance active de formule I, de 99,8 à 5 % d'un additif solide ou liquide et de 0,1 à 25 % d'un agent tensio-actif.

**24.** Procédé de préparation d'un produit selon l'une des revendications 19 à 23, caractérisé en ce que l'on mélange au moins un composé de formule I avec des additifs solides ou liquides appropriés et des agents tensio-actifs.

**25.** Procédé pour combattre ou prévenir une attaque de végétaux cultivés par des nématodes, caractérisé en ce que l'on applique sur les végétaux ou leur habitat un composé de formule I selon l'une des revendications 1 à 18.

**26.** Utilisation des composés de formule I de la revendication 1 pour le traitement et/ou la prévention d'une attaque de végétaux par des nématodes.

**27.** Utilisation selon revendication 26 de composés de formule I selon l'une des revendications 1 à 18.

**28.** Utilisation selon revendication 26 ou 27, caractérisée en ce que les nématodes appartiennent au genre Meloidogyne, Heterodera ou Globodera.